# EUROPEAN PATENT SPECIFICATION

(11) **EP 1 175 868 B1**
(45) Date of publication and mention of the grant of the patent: **30.07.2003**
(21) Application number: 01126594.9
(22) Date of filing: 17.05.1995
(51) Int. Cl.: A61B 17/115

(54) **Circular anastomosis device**
Vorrichtung für kreisformige Anastomose
Dispositif d'anastomose circulaire

(30) Priority: 26.05.1994 US 249512
(43) Date of publication of application: 30.01.2002
(62) Divisional of application: 95107521.7
(73) Proprietor: United States Surgical Corporation, Norwalk, Connecticut 06856 (US)
(72) Inventor: Robertson, John Charles, Cheshire, CT 06410 (US); Gerry, Stephen W., Bethel, CT 06801 (US); Schnut, Robert H., Carmel, NY 10512 (US); Gallagher, Richard J., Waterbury, CT 06706 (US); Fowler, David N., Trumbull, CT 06611 (US)
(74) Representative: HOFFMANN - EITLE

(56) References cited:
- EP-A- 0 270 260
- EP-A- 0 449 394
- EP-A- 0 499 457
- EP-A- 0 536 882
- EP-A- 0 537 571
- EP-A- 0 591 999
- AU-A- 5 476 486
- GB-A- 1 121 673
- GB-A- 2 070 499
- US-A- 3 593 903
- US-A- 4 485 817
- US-A- 5 104 382
- US-A- 5 137 198
- US-A- 5 271 543

## Description

### BACKGROUND

### 1. Field of the Invention

This invention relates to a surgical fastener applying instrument. More particularly, this invention relates to an arrangement for a circular anastomosis surgical stapling instrument. Such an instrument is disclosed in EP-A-449 394, which has the technical features of the pre-characterising part of claim 1 below.

### 2. Description of the Related Art

Various types of surgical fastener applying instruments have been known for the application of surgical fasteners to tissue. For example, it has been known to use various types of surgical staplers in gastric and esophageal surgery in both classic or modified gastric reconstructions performed end-to-end, end-to-side or side-to-side. In many cases, instruments, such as described in U.S. Patent No. 4,603,693, have been used where an anvil assembly mounted on the end of a center rod can be manipulated relative to a staple assembly on the end of a tubular housing of the instrument. In instruments of this nature, the center rod is connected with a mechanism, for example, which employs a wing nut at the proximal end of the instrument, so that the rod can be moved back and forth independently of the staple assembly so as to adjust the anvil assembly relative to the staple assembly. Likewise, a pusher tube is mounted within the instrument for movement via a handle mechanism so as to cause a firing of the staples from the staple assembly towards the anvil assembly.

In some instruments, such as described in U.S. Patent No. 4,351,466, these stapling instruments have been provided with a pair of handles in order to actuate the pusher tube to cause a firing of the staples. In such cases, each handle has been pivotally mounted so as to be moved toward the other handle during manual squeezing by a surgeon. Each handle also includes a lever arm within the instrument which engages against the pusher tube so as to move the tube in a proximal direction. In most surgical stapling applications, proper staple formation is very important to achieve the desired anastomosis. As such, proper alignment of parts during manufacture and precise mechanisms to control staple formation during use are advantageous.

Circular stapling instruments have also been provided with safety locks in order to prevent the squeezing together of the handles prematurely.. That is, the safety locks have been provided in order to prevent the handles from moving towards each other before a surgeon has manipulated the anvil assembly into position for the firing of the staples. While these instruments have been used safely and effectively for years, it would be advantageous to provide the feature of preventing the anvil member from being able to be moved once a fastener firing safety lock has been released. Also a continuing need exists to develop these types of surgical stapling instruments which require fewer parts and materials to manufacture, thereby reducing costs of production and requiring less labor to assemble the parts. Additionally, if the instruments are disposable, i.e. single use only, use of less materials is desirable to decrease the amount of medical waste generated during a surgical procedure.

In some applications of circular anastomosis devices, tissue must be pierced in order to connect the stapling mechanism and anvil member. U.S. Patent No. 5,119,983 discloses a removable tip portion to facilitate this procedure. After piercing tissue, the tip portion is removed and the anvil is joined to the stapler. U.S. Patent No. 5,104,025 discloses a tip portion for piercing tissue and a rigid sleeve for protecting the tip portion. The entire rigid sleeve reciprocates relative to the tip portion by means of a spring to expose the tip portion. The spring also serves to secure the sleeve to the stapling head portion of the instrument. While the removable tip embodiment of U.S. Patent No. 5,119,983 has been used successfully for years, there is always a need for more cost efficient devices.

### SUMMARY

The present invention is defined in claim 1 below, and provides a surgical fastener applying instrument which may include an anvil lockout mechanism which works in cooperation with the safety release mechanism for the fastener firing member. The instrument of the present invention can be lightweight and easy to manufacture. It need not have so many component parts as similar available instruments and, therefore, is less costly to produce.

One embodiment of the present surgical instrument includes a housing having proximal and distal end portions, a shaft extending from the housing distal end portion, the shaft having proximal and distal end portions, a fastener-carrying cartridge positioned at the shaft distal end portion, the cartridge having a plurality of fasteners disposed therein, a fastener-firing member operatively associated with the fastener-carrying cartridge, at least one lever extending from the housing, the lever being adapted to move the fastener-firing member to expel the fasteners from the cartridge, an anvil member disposed opposite the cartridge, an elongated member operatively associated with the anvil member for moving the anvil member relative to the cartridge, and locking means disposed within the housing for locking the elongated member, and, therefore the anvil member, the locking means being movable between at least a first position and a second position such that when the locking means is in the first position the elongated member is movable and when the locking means is in the second position the elongated member is prevented from moving.

In a preferred embodiment the locking means is operatively associated with a safety mechanism for preventing movement of the at least one lever, the safety mechanism being movable between at least a first position and a second position.

In another preferred embodiment, a mechanism for adjusting the distance between the fastener head assembly and the instrument housing is disclosed. This mechanism permits accurate calibration of the instrument after assembly, thereby obviating the need to closely monitor calibration during assembly and the potential need to rework assembled product.

The instrument of the invention includes a tip portion adapted to pierce tissue associated with a movable anvil retaining member. A deformable protective member is also disclosed to prevent inadvertent contact between the tip portion and tissue.

Yet another preferred embodiment includes structure disposed in the fastener head assembly for ensuring proper staple formation. The structure ensures the staples do not become over-crimped against the anvil.

### BRIEF DESCRIPTION OF THE DRAWINGS

Preferred embodiments of the invention are described hereinbelow with reference to Figures 14 to 18 of the drawings wherein:
Fig. 1 is a perspective view of an instrument for applying surgical fasteners to tissue but which lacks the hollow tissue-puncturing tip of claim 1 below;
Fig. 2 is an exploded perspective view of the instrument;
Fig. 3 is a top plan view of the fastener-firing member of the instrument;
Fig. 4 is a side plan view of the fastener-firing member;
Fig. 5 is an end view of the fastener-firing member;
Fig. 6 is a top plan view of the elongated member for moving the anvil of instrument relative to the stapling cartridge;
Fig. 7 is a side plan view of the elongated member of Fig. 6;
Fig. 8 is a cross-sectional view taken along section line 8-8 of Fig. 6;
Fig. 9 is a cross-sectional view taken along section line 9-9 of Fig. 7;
Fig. 10 is a plan view of the instrument showing the lever members in the unfired position;
Fig. 11 is a cross-sectional view taken along section line 11-11 of Fig. 10;
Fig. 11A is an enlarged view of the area indicated in Fig. 11;
Fig. 12 is a plan view of the instrument showing the lever members in the fired position;
Fig. 13 is a cross-sectional view taken along section line 13-13 of Fig. 12;
Fig. 13A is an enlarged view of the area indicated on Fig. 13;
Fig. 14 is a perspective view of an embodiment of the instrument constructed according to the present invention for applying surgical fasteners to tissue;
Fig. 15 is an exploded perspective view of the instrument of Fig. 14;
Fig. 16 is an enlarged, partially cut away cross-section view of the insert guide of the instrument of Fig. 14;
Fig. 17 is an enlarged exploded view of the distal of the instrument of Fig. 14; and
Fig. 18 is an enlarged view of the distal end of the elongate member and sharp tip of the instrument of Fig. 14.

### DETAILED DESCRIPTION OF THE PREFERRED EMBODIMENTS

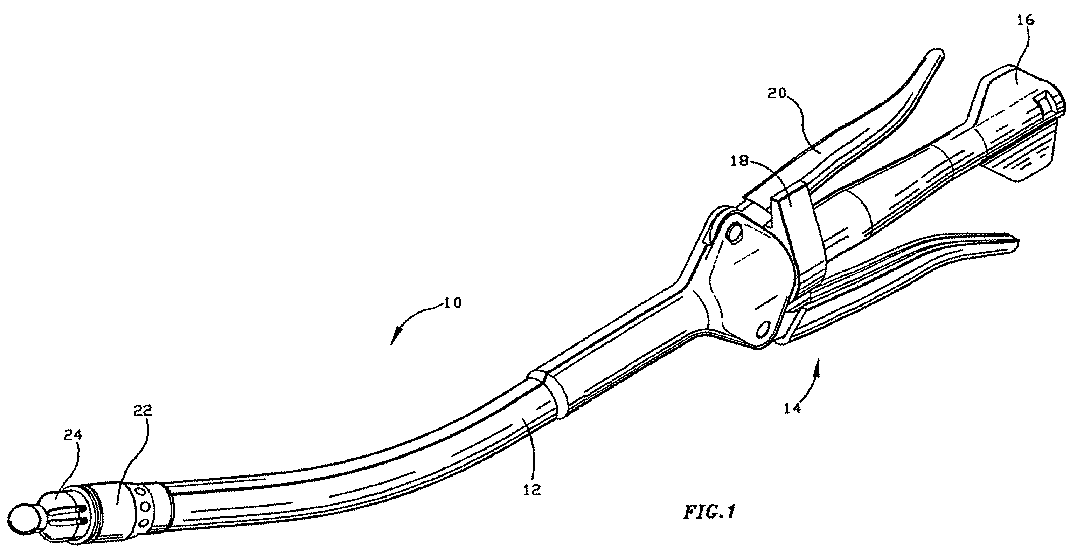

Referring now in specific detail to the drawings, in which like reference numerals identify similar or identical elements throughout the several views, and initially to Fig. 1, which shows one surgical instrument for applying a circular array of fasteners of the present invention illustrated in perspective view as instrument 10. Instrument 10 includes elongate body portion 12 and handle section 14. Handle section 14 includes anvil adjustment member 16, lever lockout or safety member 18 and fastener firing levers 20. Fastener head portion 22 and anvil member 24 are disposed at the distal end of body portion 12. Except where noted otherwise, the materials utilized in the components of the surgical instrument of the present invention generally include such materials as polycarbonate for housing sections and related components, and stainless steel for such components which transmit forces. One preferred polycarbonate material is LEXAN® brand polycarbonate available from General Electric Company. However, equivalent alternative materials will readily come to the mind of those skilled in the art.

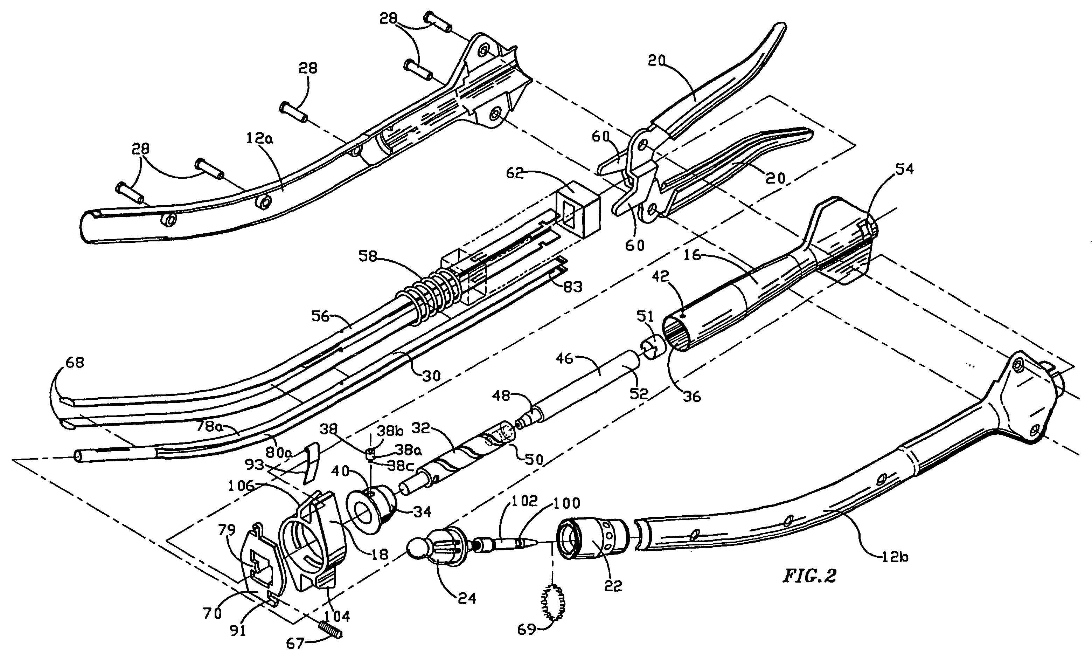

Referring now to Fig. 2, the various components of instrument 10 are shown in exploded view. Instrument 10 includes body or housing half sections 12a and 12b which are preferably molded and joined together by suitable fastening means such as rivets 28, or the like. To control axial movement of anvil member 24, elongated member 30 is slidably mounted within body portion 12, preferably by being securely mounted to helical cam member 32 by any suitable means such as, for example, welding or the like. Helical cam member 32 is slidably mounted within anvil adjustment member 16 by way of bushing 34 which is securely mounted in open end 36 of anvil adjustment member 16. Friction member 93 is disposed adjacent anvil adjustment member 16 to prevent relatively free rotation of the anvil adjustment member. In a preferred embodiment, both the mounting of bushing 34 and the camming of helical cam 32 is accomplished by compound pin 38 which has central portion 38a and extending portions 38b and 38c which are of reduced diameter. Portions 38a and 38b are press fitted into bores 40 and 42, respectively, located on bushing 34 and anvil adjustment member 16, respectively. Lower extending portion 38c serves as a camming pin and fits within the helical groove formed on the surface of helical cam 32. Anvil approximation indicator member 46 has extended portion 48 and is press fitted into proximal portion 50 of helical cam 32. Cap 51 is attached to proximal end 52 of anvil approximation indicator member 46. Cap 51 is preferably a colored piece which is easily visible through opening 54 formed near the proximal end of anvil adjustment member 16 to provide indication to the user when the anvil member is in the proper position for firing of the instrument. The distal end of elongated member 30 is provided with means to retain anvil member 24, which will be described in more detail below.

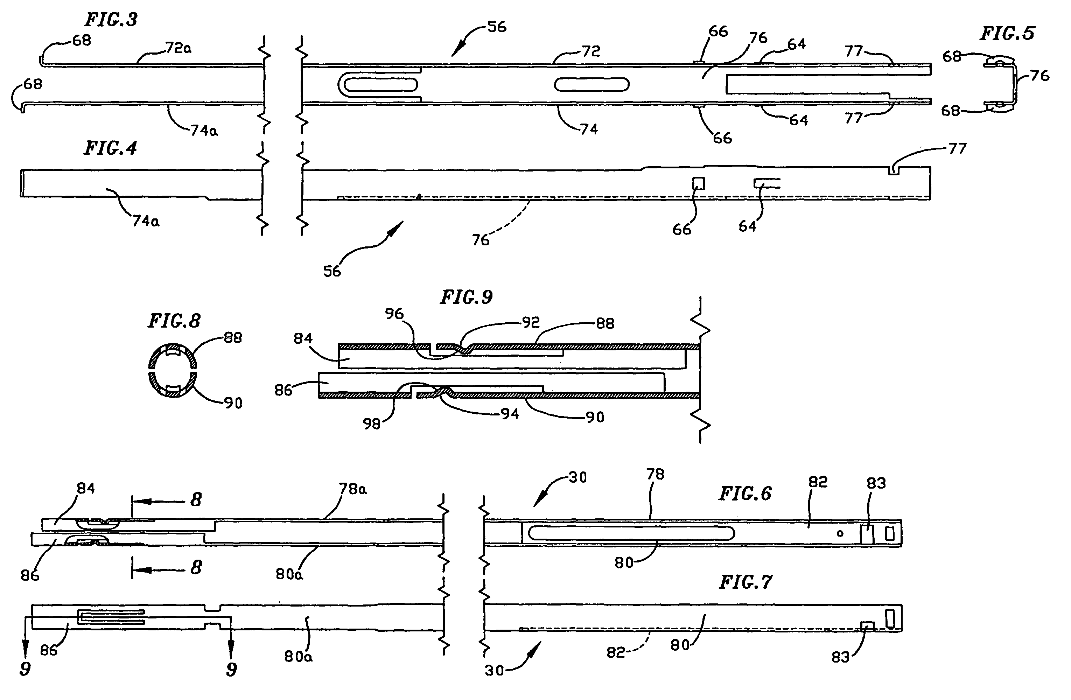

The fastener firing mechanism of instrument 10 includes fastener firing member 56 which is slidably mounted within body portion 12 preferably such that fastener firing member 56 is disposed around elongated member 30. Fastener firing member 56 is preferably biased in a proximal direction by suitable biasing means such as spring 58. Fastener firing levers 20 are pivotably mounted to body portion 12 and have extended portions 60 which cross over each other in scissor-like fashion. Bearing block 62 is mounted on fastener firing member 56, for example, being held between flexible finger portions 64 and raised portions 66 which are formed in the side walls of fastener firing member 56, as best illustrated in Figs. 3 and 4. Fastener firing member 56 has bearing surfaces such as tabs 68 formed at the distal portion which serve to urge a pusher member within fastener head portion 22 in a distal direction in order to eject surgical fasteners 69, such as stainless steel or titanium staples, from fastener head portion 22.

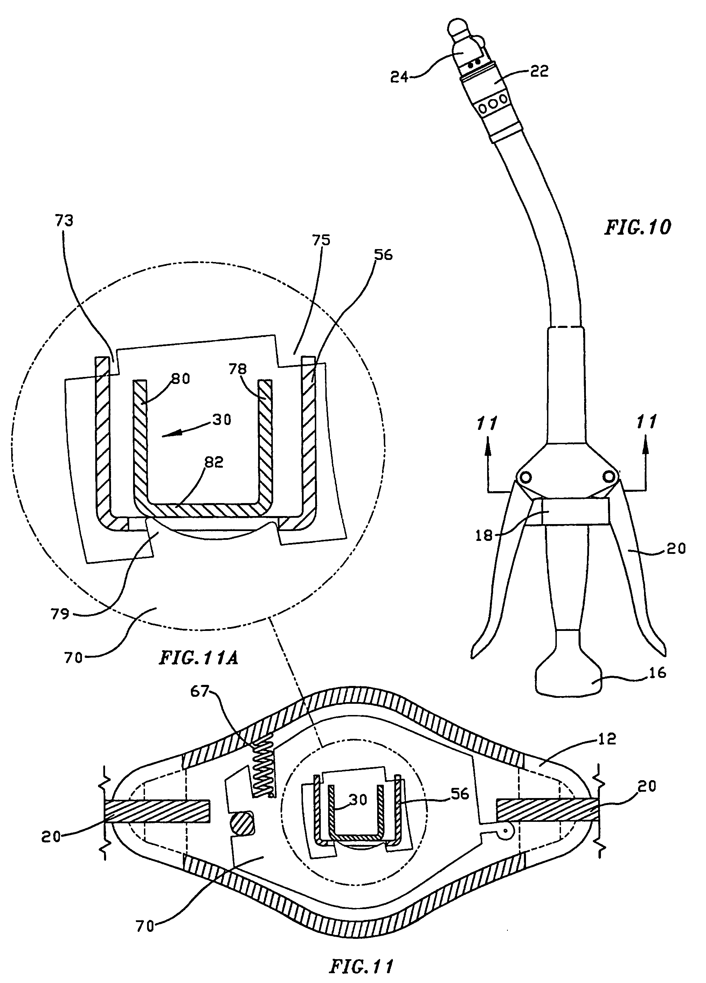

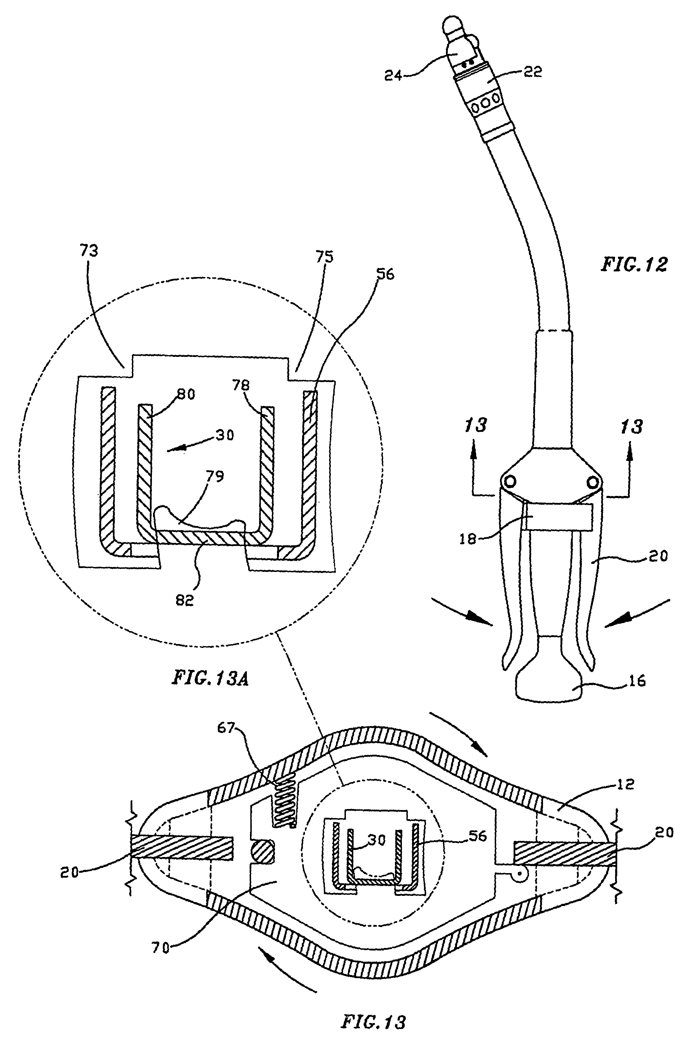

Also disposed on instrument 10 are lever lockout member 18 and elongate member lockout member 70. Lever lockout member 18 is preferably spring biased to the locked out position by spring 67 in slot 91. Each of these lockout members are preferably mounted on instrument 10 in such a manner that they are fixed relative to each other and upon pivoting of lever lockout member 18, elongate member lockout member 70 also pivots. With reference to Figs. 11A and 13A, elongate member lockout member 70 has shoulder portions 73 and 75 formed therein as well as inwardly extending tab 79. The function of each of these portions of elongate member lockout member 70 will be described in further detail later herein.

Referring now to Figs. 3-9, the structural and functional details of fastener-firing member 56 and elongated member 30 will now be described in detail. In Figs. 3-5, fastener-firing member 56 is shown as preferably being a generally U-shaped member formed from material which can transmit forces effectively and reliably such as stainless steel. Fastener-firing member 56 has side walls 72 and 74 which are connected by web 76. To fit fastener-firing member 56 within the curved section of body portion 12, fastener-firing member 56 has extended portions or flexible bands 72a and 74a which are preferably formed integrally with walls 72 and 74, respectively. Band 72a is shorter than band 74a. The difference in the length of the two bands corresponds to the amount of curvature of body portion 12 so that when fastener-firing member 56 is mounted in body portion 12, the surfaces of tabs 68 form a plane parallel with the surface of the fastener pusher member (not shown ) .

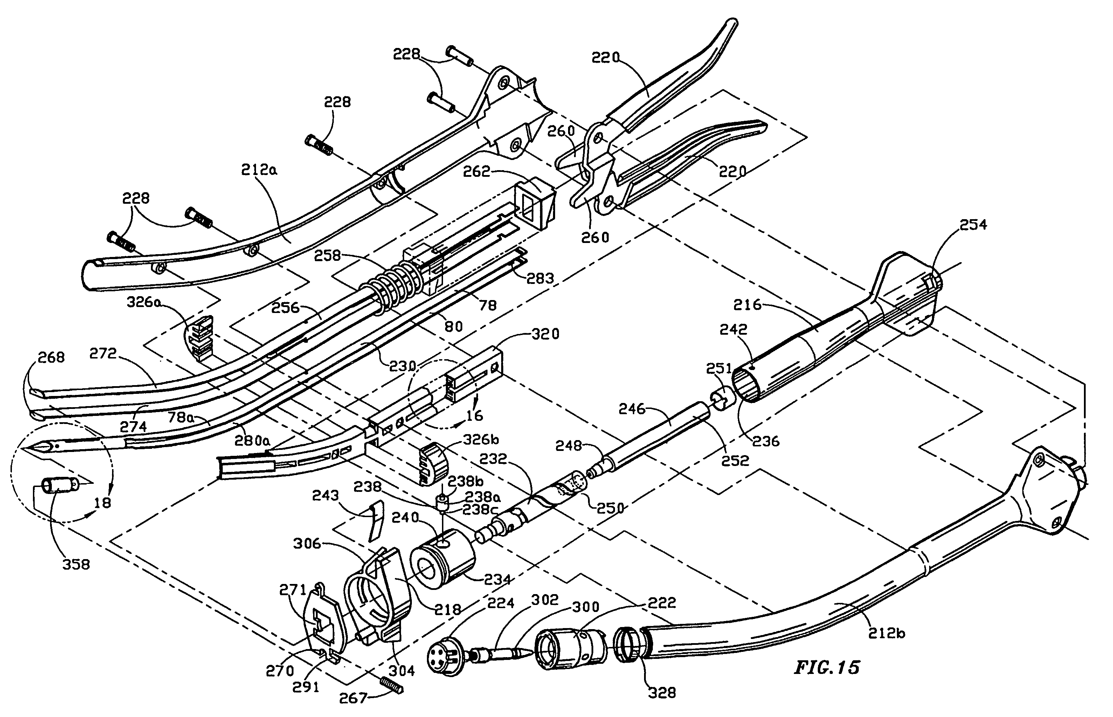

Referring to Figs. 6-9, elongated member 30 is shown as a U-shaped member, similar to fastener-firing member 56. Elongated member 30 has side walls 78 and 80 which are joined by web 82. However, the cross-section dimensions of elongated member 30 are preferably such that elongated member 30 readily fits within fastener-firing member 56. This arrangement is desirable so that elongated member 30 and fastener-firing member 56 can slide independent of each other. As with fastener-firing member 56, elongated member 30 must also be formed to fit within the curved contour of body portion 12. To accomplish this curvature, elongated member 30 has extended portions or flexible bands 78a and 80a which are preferably formed integrally with walls 78 and 80, respectively. Similar to the construction of fastener-firing member 56, bands 78a and 80a of elongated member 30 are of different length. Elongated member 30 terminates at a distal end in a pair of opposed anvil-retaining portions 84 and 86. Preferably, structure can be provided within body portion 12 (see 320 in Fig. 15) that serves to retain the side walls and bands of both elongated member 30 and fastener-firing member 56. Such structure can be of unitary construction and have grooves to direct longitudinal movement of the channels and bands. Additionally, one or more seals (see 326a dn 326b in Fig. 15) can be disposed within body portion 12 to prevent the flow of gases therethrough.

To facilitate retaining anvil member 24, and in particular, the anvil shaft therein, anvil-retaining portions 84 and 86 are preferably semi-circular in shape as best illustrated in the cross-section view of Fig. 8. To assist in retention of anvil 24, anvil-retaining portions 84 and 86 are provided with flexible finger portions 88 and 90, respectively, each of which have a raised portion formed thereon, such as camming and retaining portions 92 and 94, respectively. Camming surfaces 96 and 98 formed by camming and retaining portions 92 and 94, respectively serve to cam flexible finger portions 88 and 90 radially outward upon insertion of the anvil into the distal end of instrument 10. Referring temporarily back to Fig. 2, once annular groove portion 100 of anvil shaft 102 passes between retaining portions 84 and 86, flexible finger portions 88 and 90 return toward their initial or at rest state so that retaining portions 92 and 94 seat in annular groove 100.

In a preferred embodiment, extended portions 78a and 80a of elongated member 30 are preferably bent until the ends of anvil-retaining portions 84 and 86 are aligned and are then permanently joined together (as shown in Fig. 2), by suitable bonding techniques, such as, by welding.

At the proximal end of elongated member 30, cut out portion 83 is formed to receive elongate member lockout member 70 when elongated member is properly positioned for firing the staples of instrument 10. As best illustrated in Figs. 6 and 7,cut-out portion 83 is preferably formed through most of web 82 and continues partially up side wall 80.

The basic steps of operation are set forth in several patents, such as U.S. Patents 4,576,167 issued to Noiles, 5,005,749 issued to Aranyi, and 5,119,983 to Green et al.

With reference to the illustrated instrument the user positions the tissue to be joined between anvil 24 and fastener head portion 22. Anvil adjustment member 16 is rotated to move elongated member 30 and anvil 24 proximally until the user sees approximation indicator 46 appear in opening 54 of anvil adjustment member 16. During this step, elongate member 30 acts as a tension member as it pulls anvil 24 into position adjacent fastener head portion 22. Prior to alignment of cut-out 83 in elongate member 30 and extended portion 79 of elongate member lockout 70, lockout 70 is prevented from pivoting by contact between extended portion 79 and elongate member 30. When cut out 83 is positioned adjacent extended portion 79 of elongate member lockout 70, as further described below, lever lockout member 18 and elongate member lockout 70 are able to be pivoted by depressing, usually with the thumb, on lever lockout member 18. Members 18 and 70 can be interconnected by any suitable means, i.e., protrusion from one member entering a recess in the other. Once lever lockout member 18 is pivoted by the user, fastener-firing levers 20 are depressed to urge fastener-firing member 56 in a distal direction. This motion is accomplished by the camming effect of extended portions 60 of fastener-firing levers 20 on bearing block 62 the distal movement of fastener-firing member 56 urges fastener pusher members to eject fasteners 69 from fastener head portion 22. During this step, fastener-firing member 56 acts as a compression member as it ejects fasteners 69.

With the above operational description of instrument 10 as a general base of the overall operation, the operation of elongate member lockout 70 will now be described in detail with reference to Figs. 10-13. Once the user has instrument 10 inserted and the tissue to be joined is properly situated about the distal end of the instrument, anvil 24 is approximated to its proper position by rotation of anvil adjustment member 16, instrument 10 is positioned for firing, as shown in Fig. 10. In that position, however, fastener- firing member 56 is still blocked from movement due to lever lockout member 18 still being oriented in the "safety on" position, *i.e*., lever locking extended portions 104 and 106 (Fig. 2) are aligned with the structure of levers 20 so that they cannot be depressed. Pivoting of lever lockout member 18 is prevented when elongated member 30 is out of the desired approximation range for firing the staples.

With reference to Figs. 2-7, 11 and 13, prevention of the ability to pivot lever lockout member 18 is accomplished by the relative position of elongated member 30 and thus the approximation of anvil 24. When anvil 24 is not properly approximated, side wall 80 and web 82 of elongated member 30 prevent extended portion 79 of elongate member lockout 70 from moving further inward (Fig. 11). However, once elongated member 30 is properly positioned, *i*.*e*., cut-out 83 is aligned with extended portion 79 of elongate member lockout 70, then lever lockout member 18 which is fixedly secured to elongate member lockout member 70, can be pivoted, as shown in Fig. 13. When lockout 70 is pivoted, shoulder portions 73 and 75 of lockout 70 are moved out of notches 77 of fastener-firing member 56 (Figs. 3 and 4). This enables fastener-firing levers 20 to be pivoted toward each other as shown in Fig. 12, thereby moving fastener-firing member 56 distally and ejecting fasteners 69 from fastener head portion 22. As can be seen in Figs. 13 and 13A once lever lockout 18, and elongate member lockout 70 are pivoted by the user, inwardly extending portion 79 of lockout 70 enters into cut-out 83 (Figs. 6 and 7) and blocks elongated member 30 from movement in either a proximal or distal direction.

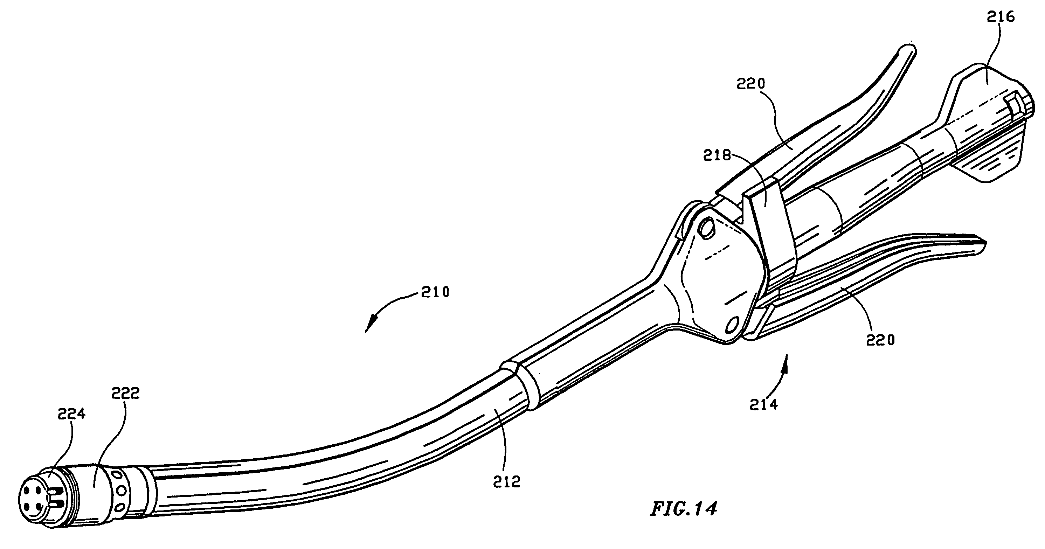

Referring now to Figs. 14-18, a first embodiment of the present invention shown as instrument 210 will now be described. For purposes of clarity, components of the instrument of Figs. 14-18 which are similar or identical to those of the instrument shown in Figs. 1-13 have been labeled by adding 200 to the reference numerals of the instrument described above. For example, anvil shaft 102 of Fig. 2 corresponds to anvil shaft 302 of Fig. 15.

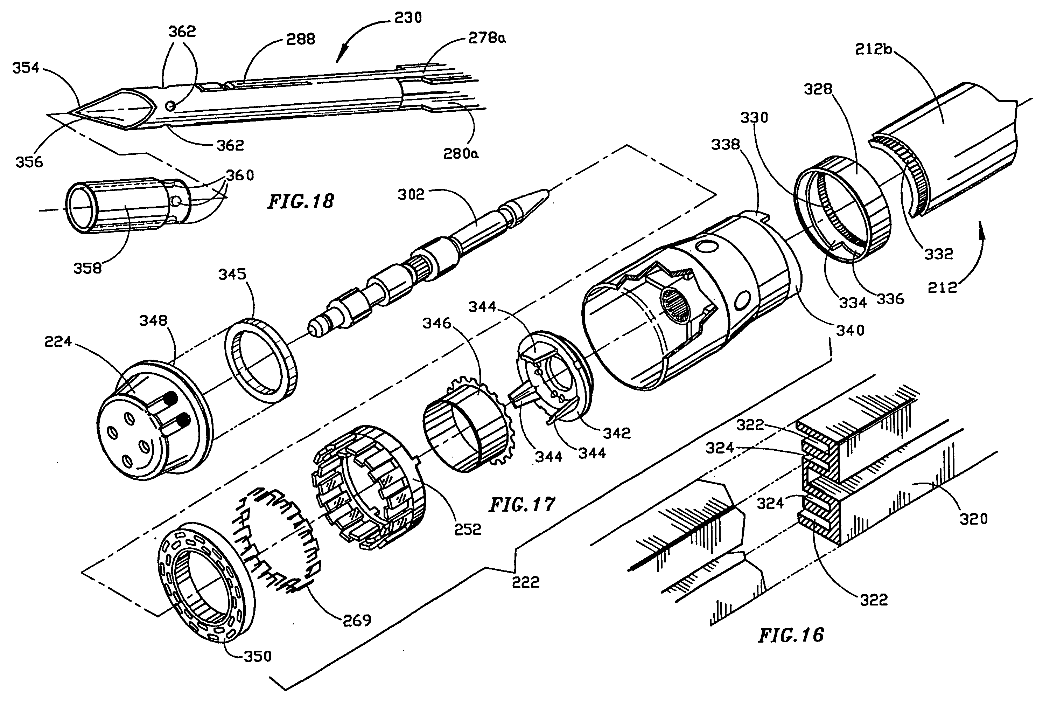

Referring now to Fig. 14 in conjunction with Fig. 15, instrument 210 includes insert guide 320 which is preferably molded of a polycarbonate material such as LEXAN® available from the General Electric Company. Insert guide 320 is preferably molded to conform to the curvature of housing half-sections 212a and 212b so as to provide structural support for elongate member 230 and fastener-firing member 256. Referring temporarily to Fig. 16, insert guide 320 is illustrated in a partially cut-away section to show the guide tracks which receive elongate member 230 and fastener-firing member 256 therein. Specifically, outer guide tracks 322 are molded to receive sidewalls 272 and 274 therein and inner guide tracks 324 are formed to receive side walls 78 and 80 of elongate member 30 therein. Insert guide 320 thereby provides structural support upon longitudinal motion of either elongate member 230 or fastener-firing member 256, particularly at the point of curvature thereof. Insert guide 320 effectively reduces possible force transmission losses upon operation of instrument 210.

In order to provide an internal seal in body portion 212, seal half-sections 326a and 326b are formed to fit around notches formed on insert guide 320. Seal half-sections 326a and 326b are preferably molded of a hard rubber material which is molded to conform with the inner dimensions of housing half-sections 212a and 212b. Additionally, seal half-sections 326a and 326b are molded to fit over elongate member 230 and fastener-firing member 256 so as to form a seal between the internal section of the instrument 210 which is distal of the seal half-sections 326a and 326b and the internal portions of instrument 210 which are proximal of seal half-sections 326a and 326b so as to inhibit the passage of gases thereby.

Referring now to figure 15 in conjunction with Fig. 17, additional features of the present invention are illustrated therein which will now be described. An adjusting member such as collar 328 is mounted between the distal end of housing 212 and the proximal end of fastener head portion assembly 222. Collar 328 has ratchet teeth 330 formed on an inner surface thereof at the proximal end. Ratchet teeth 330 cooperate with ratchet teeth 332 formed on the distal end of housing half-sections 212a and 212b. Ratchet teeth 330 and 332 interact to provide for rotational advancement of collar 328 in a single direction. Also formed on collar 328 are camming surfaces 334 and 336 which interact with camming surfaces 338 and 340 formed at the proximal end of fastener head portion 222. A purpose of collar 328 is to provide for post instrument assembly adjustment of the relative longitudinal positioning of fastener head portion assembly 222 with respect to the distal end of body portion 212. This adjustment capability is important so that instrument 210 may be calibrated to insure the proper positioning of anvil member 224 when cap 251 of anvil approximation indicator member 246 is positioned within opening 254 so as to indicate that instrument 210 is ready for firing of surgical staples 269. Once instrument 210 is properly calibrated, collar member 328 may be fixedly secured to the distal end of body portion 212 so that collar 328 is not permitted to rotate with respect thereto. This attachment may be accomplished by known suitable means such as, for example, bonding and/or by the use of a fastener such as a screw or a pin.

Another important feature illustrated in Fig. 17 provides protection against over-crimping of surgical staples 269 upon firing of instrument 210. Pusher back 342 is provided with longitudinally extending portions 344 which are configured to fit inside of knife ring 346 and are preferably sized to abut against a surface portion, such as cut ring 345, of anvil 224 upon firing of instrument 210. When portions 344 of pusher back 342 contact a portion of anvil 224, pusher fingers 252 are prevented from traveling further, thereby limiting the degree to which surgical staples 269 may be crimped against anvil 224.

In another feature of the present invention, referring now Fig. 15 in conjunction with Fig. 18, elongate member 230 has tip 354 formed at the distal end to facilitate puncturing of tissue as desired by the user of instrument 210. Tip 354 is preferably sharp and formed by known methods such as, for example, grinding with suitable abrasives. Tip 354 is formed such that a hollow passageway or opening 356 is formed which passes longitudinally through the distal end portion of elongate member 230. Opening 356 on elongate member 230 is preferably sized so that it can receive shaft 302 of anvil 224 so that anvil 224 may be retained by the distal end portion of elongate member 230 in a similar manner as set forth above for elongate member 30 and anvil 24 for the embodiment of Figs. 1-13. To avoid inadvertent lateral contact with tip 354 a protective cover is provided, such as cover 358 which is preferably formed of a deformable elastomeric material. Cover 358 is provided with raised portions 360 which correspond to bores 362 formed on elongate member 230 proximal of tip 354. In use, when elongate member 230 is extended past head portion assembly 222, cover 358 deflects upon contact with tissue to expose tip 354. After tip 354 and cover 358 pass through the tissue, cover 358 returns to a position which protects tip 354, thereby providing means for preventing inadvertent trauma by tip 354 to surrounding tissue.

The operation of instrument 210 is similar to that of instrument 10 except as noted hereinabove. Accordingly, no separate description of the operation of instrument 210 will be provided at this point.

The claims which follow reveal embodiments of the invention additional to those described in detail above.

## Claims

1. A surgical instrument (210) for applying at least one circular array of fasteners (269), comprising:
a housing (214) having proximal and distal portions;
an elongated shaft (212) extending from the housing distal portion, the shaft having proximal and distal end portions;
a fastener-carrying cartridge (222) positioned at the shaft distal end portion; and
an elongated anvil-retaining member (230) reciprocatingly movable within the fastener-carrying cartridge; an anvil member (224); the instrument being **characterised by**:
a hollow distal portion (356) forming a tissue-puncturing tip (354) at a distal end of the anvil-retaining member; and wherein
the anvil member (224) is adapted for removable attachment to the elongate anvil-retaining member through a shaft (302) that is received within the said hollow distal portion.

2. A surgical instrument as claimed in claim 1, further comprising an adjusting member (328) operatively associated with the fastener-carrying cartridge to adjust the longitudinal positioning of the fastener-carrying cartridge relative the distal end of the shaft.

3. A surgical instrument as recited in claim 2, wherein the adjusting member includes at least one camming surface (334, 336) operatively associated with the fastener-carrying cartridge.

4. A surgical instrument as recited in claim 3, wherein the fastener-carrying cartridge includes at least one camming surface (338, 340) operatively associated with the at least one camming surface of the adjusting member.

5. A surgical instrument as recited in claim 2 or 3, wherein the adjusting member is a rotatable collar member.

6. A surgical instrument as recited in claim 5, wherein the collar member includes incremental portions (330) disposed thereon adapted to incrementally facilitate movement of the collar member camming surface upon movement of the collar member relative to the distal end of the elongated shaft.

7. A surgical instrument as recited in claim 6, wherein the incremental portions of the collar member are adapted to facilitate movement of the collar member in a single direction.

8. A surgical instrument as recited in claim 6 or 7, wherein the incremental portions of the collar member include a plurality of ratchet teeth which are operatively associated with at least one tooth portion (332) formed on the elongated shaft.

9. A surgical instrument as recited in any one of the preceding claims, further comprising a resilient shield member (358) connected to the elongate anvil-retaining member and disposed around the tip.

10. A surgical instrument as recited in claim 9, wherein the resilient shield member is at least partially formed from an elastomeric material.

11. A surgical instrument as claimed in any one of the preceding claims, comprising:
a pusher member (252) operatively associated with surgical fasteners within the cartridge, a knife member (346) disposed within the cartridge and a fastener crimping limit member (342) disposed within the cartridge.

12. A surgical instrument as claimed in claim 11, wherein the fastener crimping limit member (342) includes a base having a longitudinal portion (344) extending therefrom which is operatively associated with the anvil member to limit the crimping of the surgical fasteners upon firing of the instrument.

13. A surgical instrument as claimed in claim 12, wherein the longitudinally extending portion comprises finger portions (344).

## Patentansprüche

1. Chirurgisches Instrument (210) zum Anbringen zumindest eines kreisförmigen Feldes von Befestigern (269), mit:
einem Gehäuse (214) mit einem proximalen und einem distalen Bereich;
einem länglichen Schaft (212), der sich von dem distalen Bereich des Gehäuses aus erstreckt und einen proximalen und einen distalen Endbereich hat;
einer Befestiger tragenden Patrone (222), die sich am distalen Endbereich des Schafts befindet;
einem länglichen Amboß-Rückhalteelement (230), das innerhalb der Befestiger tragenden Patrone hin- und herbeweglich ist; und
einem Ambosselement (224);
wobei das Instrument (210) **gekennzeichnet ist durch**:
einen hohlen distalen Bereich (356), der eine Gewebe durchstechende Spitze (354) an einem distalen Ende des Ambossrückhalteelements ausbildet, wobei das Ambosselement (224) lösbar an das längliche Ambossrückhalteelement **durch** einen Schaft (302) anbringbar ist, der in dem hohlen distalen Bereich aufgenommen ist.

2. Chirurgisches Instrument nach Anspruch 1, weiter mit einem Einstellelement (328), das operativ der Befestiger tragenden Patrone zugeordnet ist, um die Längsposition der Befestiger tragenden Patrone relativ zum distalen Ende des Schafts einzustellen.

3. Chirurgisches Instrument nach Anspruch 2, wobei das Einstellelement zumindest eine Nockenfläche (334, 336) beinhaltet, die operativ der Befestiger tragenden Patrone zugeordnet ist.

4. Chirurgisches Instrument nach Anspruch 3, wobei die Befestiger tragende Patrone zumindest eine Anschlagfläche (338, 340) beinhaltet, die operativ der zumindest einen Nockenfläche des Einstellelements zugeordnet ist.

5. Chirurgisches Instrument nach Anspruch 2 oder 3, wobei das Einstellelement ein drehbares Kragenelement ist.

6. Chirurgisches Instrument nach Anspruch 5, wobei das Kragenelement stufenweise Bereiche (330) beinhaltet, die daran angeordnet sind und die schrittweise Bewegung der Anschlagfläche des Kragenelements bei einer Bewegung des Kragenelements relativ zum distalen Ende des länglichen Schafts erleichtern.

7. Chirurgisches Instrument nach Anspruch 6, wobei die stufenweisen Bereiche des Kragenelements die Bewegung des Kragenelements in einer einzigen Richtung erleichtern.

8. Chirurgisches Instrument nach Anspruch 6 oder 7, wobei die stufenweisen Bereiche des Kragenelements mehrere Ratschenzähne beinhalten, welche operativ zumindest einem Zahnbereich (332) an dem länglichen Schaft zugeordnet sind.

9. Chirurgisches Instrument nach einem der vorangehenden Ansprüche, weiter mit einem nachgiebigen Schirmelement (358), das mit dem länglichen Ambossrückhalteelement verbunden und um die Spitze herum vorgesehen ist.

10. Chirurgisches Instrument nach Anspruch 9, wobei das nachgiebige Schirmelement zumindest teilweise aus einem elastomerischen Material besteht.

11. Chirurgisches Instrument nach einem der vorangehenden Ansprüche, mit einem Drückelement (252), das chirurgischen Befestigern innerhalb der Patrone operativ zugeordnet ist, einem Messerelement (346) innerhalb der Patrone sowie einem Element (342) zum Begrenzen des Crimpens der Befestiger, welches Element (342) innerhalb der Patrone vorgesehen ist.

12. Chirurgisches Instrument nach Anspruch 11, wobei das Element (342) zum Begrenzen des Crimpens der Befestiger eine Basis mit einem längs verlaufenden Bereich (344) beinhaltet, die sich von dort aus erstreckt, welche operativ dem Ambosselement zugeordnet ist, um das Crimpen der chirurgischen Befestiger beim Feuern des Instruments zu begrenzen.

13. Chirurgisches Instrument nach Anspruch 12, wobei der längs verlaufende sich erstreckende Bereich Fingerbereiche (344) aufweist.

## Revendications

1. Instrument chirurgical (210) pour appliquer au moins une rangée circulaire d'attaches (269), comprenant :
un boîtier (214) présentant des portions proximale et distale ;
une tige oblongue (212) s'étendant à partir de la portion distale du boîtier; la tige présentant des portions d'extrémité proximale et distale ;
une cartouche porteuse d'attaches (222) positionnée à la portion d'extrémité distale de la tige ; et
un élément oblong de retenue d'enclume (230) déplaçable selon un mouvement alternatif dans la cartouche porteuse d'attaches ;
un élément d'enclume (224) ;
l'instrument étant **caractérisé par** :
une portion distale creuse (356) formant une pointe (354) ponctionnant le tissu à une extrémité distale de l'élément de retenue d'enclume ; et où
l'élément d'enclume (224) est conçu pour une fixation amovible à l'élément oblong de retenue d'enclume par une tige (302) qui est reçue dans ladite portion distale creuse.

2. Instrument chirurgical selon la revendication 1, comprenant en outre un élément d'ajustement (328) fonctionnellement associé à la cartouche porteuse d'attaches pour ajuster le positionnement longitudinal de la cartouche porteuse d'attaches relativement à l'extrémité distale de la tige.

3. Instrument chirurgical selon la revendication 2, où l'élément d'ajustement comporte au moins une surface de came (334, 336) fonctionnellement associée à la cartouche porteuse d'attaches.

4. Instrument chirurgical selon la revendication 3, où la cartouche porteuse d'attaches comporte au moins une surface de came (338, 340) fonctionnellement associée à au moins une surface de came de l'élément d'ajustement.

5. Instrument chirurgical selon la revendication 2 ou 3, où l'élément d'ajustement est un élément de collier tournant.

6. Instrument chirurgical selon la revendication 5, où l'élément de collier comporte des portions incrémentielles (330) disposées sur celui-ci aptes à faciliter un mouvement par incréments de la surface de came de l'élément de collier lors d'un mouvement de l'élément de collier relativement à l'extrémité distale de la tige oblongue.

7. Instrument chirurgical selon la revendication 6, où les portions incrémentielles de l'élément de collier sont conçues pour faciliter le mouvement de l'élément de collier dans une seule direction.

8. Instrument chirurgical selon la revendication 6 ou 7, où les portions incrémentielles de l'élément de collier comportent plusieurs dents de rochet qui sont fonctionnellement associées à au moins une portion dentée (332) formée sur la tige oblongue.

9. Instrument chirurgical selon l'une des revendications précédentes, comprenant en outre un élément de protection élastique (358) relié à l'élément oblong de retenue d'enclume et disposé autour de la pointe.

10. Instrument chirurgical selon la revendication 9, où l'élément de protection élastique est au moins partiellement réalisé en un matériau élastomère.

11. Instrument chirurgical selon l'une des revendications précédentes, comprenant :
un élément de poussée (252) fonctionnellement associé aux attaches chirurgicales dans la cartouche, un élément formant couteau (346) disposé dans la cartouche et un élément (342) limitant le rabattement de l'attache, disposé dans la cartouche.

12. Instrument chirurgical selon la revendication 11, où l'élément (342) limitant le rabattement de l'attache comporte une base avec une portion longitudinale- (344) s'étendant à partir de celle-ci qui est fonctionnellement associée à l'élément d'enclume pour limiter le rabattement des attaches chirurgicales lors du tir avec l'instrument.

13. Instrument chirurgical selon la revendication 12, où la portion s'étendant longitudinalement comprend des portions de doigt (344).
